(19) Europäisches Patentamt
European Patent Office
Office européen des brevets

(11) EP 1 997 838 A2

(12) DEMANDE DE BREVET EUROPEEN

(43) Date de publication:
**03.12.2008 Bulletin 2008/49**

(51) Int Cl.:
***C08F 220/04*** *(2006.01)*

(21) Numéro de dépôt: **08013582.5**

(22) Date de dépôt: **11.06.1993**

(84) Etats contractants désignés:
**AT BE CH DE DK ES FR GB GR IT LI NL SE**

(30) Priorité: **01.07.1992 FR 9208399**

(62) Numéro(s) de document de la (des) demande(s) initiale(s) en application de l'article 76 CBE:
**02076892.5 / 1 270 617**
**93420239.1 / 0 577 526**

(27) Demande déposée antérieurement:
**11.06.1993 EP 02076892**

(71) Demandeur: **COATEX S.A.S.**
**69730 Genay (FR)**

(72) Inventeurs:
- **Egraz, Jean-Bernard**
  **69130 Ecully (FR)**
- **Grondin, Henri**
  **69580 Sathonay Village (FR)**
- **Suau, Jean-Marc**
  **69480 Lucenay (FR)**

Remarques:
Cette demande a été déposée le 29-07-2008 comme demande divisionnaire de la demande mentionnée sous le code INID 62.

(54) **Copolymere acrylique partiellement ou totalement hydrosoluble, reticule ou non et son utilisation**

(57) L'invention consiste en des compositions aqueuses chargées et/ou pigmentées contenant un copolymère constitué d'au moins un monomère à insaturation éthylénique et à fonction carboxylique, éventuellement d'au moins un monomère à insaturation éthylénique et sans fonction carboxylique, d'au moins un monomère oxyalkylé à insaturation éthylénique et terminé par une chaîne grasse hydrophobe possédant au moins 26 atomes de carbone et d'éventuellement au moins un monomère possédant au moins deux insaturations éthyléniques.

EP 1 997 838 A2

## Description

La présente invention se rapporte à un nouveau copolymère partiellement ou totalement hydrosoluble réticulé ou non et constitué de motifs monomériques distincts composés d'au moins un monomère à insaturation éthylénique et à fonction carboxylique, éventuellement au moins un monomère à insaturation éthylénique et sans fonction carboxylique, au moins un monomère oxyalkylé à insaturation éthylénique et terminé par une chaîne grasse hydrophobe possédant au moins 26 atomes de carbone et éventuellement au moins un monomère possédant au moins deux insaturations éthyléniques.

La présente invention concerne également l'application de ce copolymère comme modificateur de rhéologie dans des applications aussi diverses que les boues de forage, les pâtes d'impression textile, la cosmétique, ou encore la détergence, et autres compositions de revêtement comme la peinture et comme agent antisédimentation et/ou de mise en suspension pour des charges minérales ou organiques grossières dans divers domaines comme par exemple le phytosanitaire.

On connait déjà des copolymères formés de motifs monomériques dont le premier est carboxylé et le deuxième non.

Ainsi, le brevet EP 0 173 109 décrit un copolymère constitué d'acide carboxylique à insaturation éthylénique, d'ester à insaturation éthylénique et d'un troisième monomère produit de la réaction d'un alcool gras contenant 6 à 22 atomes de carbone avec un isocyanate insaturé.

De même, le brevet EP 0 013 836 révèle, mais seulement de manière très générale, un copolymère à base d'acide méthacrylique, d'acrylate d'alkyle ayant jusqu'à 4 atomes de carbone et d'un monomère oxyalkylé terminé par une chaîne grasse ayant jusqu'à 30 atomes de carbone, tout comme le brevet EP 0 011 806 qui décrit également le même type de copolymère dont le troisième monomère est un monomère à insaturation éthylénique et terminé par une chaîne grasse possédant jusqu'à 20 atomes de carbone, ou encore le brevet EP 0 248 612 qui revendique un copolymère dont le troisième monomère est un ester dont la chaîne grasse contient jusqu'à 25 atomes de carbone.

On connaît également un autre brevet EP 0 216 479 qui décrit, mais seulement de manière très générale, un copolymère à base de monomère ionique à insaturation éthylénique, de monomère substantiellement non ionique à insaturation éthylénique, d'un éther allylique oxyalkylé ayant jusqu'à 30 atomes de carbone et d'un réticulant.

Mais toute cette littérature décrit des polymères dont l'utilisation dans certaines compositions aqueuses ne donne pas entière satisfaction.

Le problème que la présente invention vise à résoudre consiste à augmenter l'épaississement de compositions aqueuses sous bas gradient de cisaillement grâce à l'efficacité épaississante accrue des polymères selon l'invention et à conférer aux suspensions aqueuses de matières minérales ou organiques des stabilités remarquables, notamment sur le plan de l'antisédimentation sans qu'il y ait pour autant d'augmentation notable des viscosités comme c'est souvent le cas des polymères décrits dans l'art antérieur.

Ces comportements très spécifiques des polymères de l'invention sont apportés par le choix et la nature des motifs monomériques constituants les macromolécules.

L'homme du métier saura envisager des produits équivalents aux produits décrits dans la présente demande notamment en faisant varier dans le troisième monomère le nombre de moles d'oxyde d'alkylène condensé, et la longueur de la chaîne grasse hydrophobe de façon à conserver des propriétés équivalentes aux produits exemplifiés. L'homme du métier pourra notamment préconiser le nombre d'oxyde d'alkylène en fonction du nombre de carbone de la chaîne grasse et en fonction de l'application.

Dans un souci de clarté dans toute la présente demande il ne sera fait référence qu'au nombre de carbone de la chaîne grasse hydrophobe du monomère spécial oxyalkylé.

La présente invention a donc d'abord pour objet un copolymère de formule générale :

$$(A)_a\text{---}(B)_b\text{---}(C)_c\text{---}(D)_d$$

dans laquelle :

(A) représente le ou les monomères à insaturation éthylénique et à fonction carboxylique avec "a" le pourcentage pondéral du monomère (A) par rapport au poids total des monomères,

(B) représente le ou les monomères à insaturation éthylénique et sans fonction carboxylique avec "b" le pourcentage pondéral du monomère (B) par rapport au poids total des monomères, "b" pouvant être nul.

(C) représente le monomère oxyalkylé à insaturation éthylénique et terminé par une chaîne grasse hydrophobe possédant au moins 26 atomes de carbone avec "c" le pourcentage pondéral du monomère (C) par rapport au poids total des monomères.

Le monomère (C) sera nommé dans la suite de la description "monomère spécial" et s'écrit suivant la formule (I) suivante :

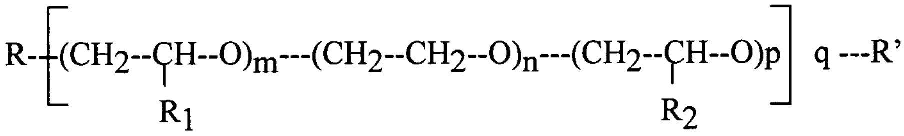

dans laquelle :

- m et p représentent un nombre de motifs d'oxyde d'alkylène,
- n représente un nombre de motifs d'oxyde d'éthylène,
- q un nombre au moins égal à 1 et tels que :

$$q(n + m + p) \leq 100,$$

- R le radical insaturé polymérisable,
- R' le radical hydrophobe à chaîne grasse possédant au moins 31 atomes de carbone,
- R1 l'hydrogène ou le groupe méthyle,
- R2 l'hydrogène ou le groupe méthyle.

(D) représente le ou les monomères possédant au moins deux insaturations éthyléniques avec "d" le pourcentage pondéral du monomère (D) par rapport au poids total des monomères, "d" pouvant être nul et tel que a + b + c + d = 100 %.

Ainsi, alors que l'art antérieur décrit de très nombreux copolymères à motifs monomériques distincts donnant aux compositions aqueuses un comportement rhéologique aboutissant à des augmentations notables des viscosités, le copolymère selon l'invention s'en distingue par le fait qu'il est constitué :

a. d'au moins un monomère à insaturation éthylénique et à fonction carboxylique choisi parmi les monoacides, tels que l'acide acrylique, méthacrylique, crotonique, isocrotonique, cinnamique, les diacides, tels que l'acide itaconique, fumarique, maléïque, citraconique, les anhydrides d'acides carboxyliques, tels que l'anhydride maléïque et les hémiesters de diacides, tels que les monoesters en $C_1$ à $C_4$, des acides maléïque ou itaconique.
Toutefois, le monomère éthylénique carboxylé est préférentiellement choisi dans le groupe constitué par les acides acrylique, méthacrylique et itaconique,

b. éventuellement d'au moins un monomère à insaturation éthylénique et sans fonction carboxylique, choisi d'une manière non limitative dans le groupe constitué par les esters d'acides acrylique ou méthacrylique, tels que les acrylates ou méthacrylates de méthyle, éthyle, butyle, 2-éthyl-hexyle, ou encore parmi l'acrylonitrile, l'acétate de vinyle, le styrène, le méthylstyrène, le diisobutylène, la vinylpyrolidone, la vinylcaprolactame.
Cependant, le monomère à insaturation éthylénique sans fonction carboxylique est préférentiellement choisi parmi les esters acryliques, tels que les acrylate et méthacrylate d'alkyle en $C_1$ à $C_4$.

c. d'au moins un monomère appelé "monomère spécial" oxyalkylé à insaturation éthylénique et terminé par une chaîne grasse hydrophobe, de formule générale (I) :

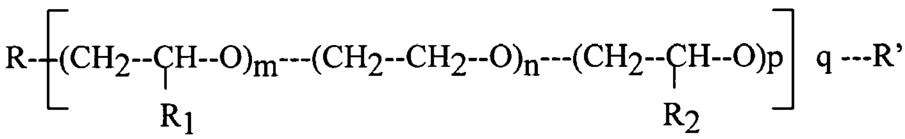

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$q(n + m + p) \leq 100,$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α diméthyl-m-isopropenylbenzyluréthanne, allyluréthanne ou bien encore les éthers allyliques, les acrylamide et méthacrylamide substituées ou non, des vinyliques.
R' représente le radical hydrophobe à chaîne grasse tels que les groupes linéaire ou ramifié alkyle, alkylaryle, arylalkyle, aryle ayant au moins 31 atomes de carbone.

d. éventuellement d'au moins un monomère possédant au moins deux insaturations éthyléniques choisi d'une manière non limitative dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols tels que le pentaérythritol, le sorbitol, le sucrose ou autres.

Le copolymère selon l'invention, obtenu par des procédés connus de copolymérisation radicalaire en solution, en émulsion directe ou inverse, en suspension ou précipitation dans des solvants appropriés, en présence de systèmes catalytiques et d'agents de transfert connus, contient préférentiellement, exprimé en pour cent en poids :

a. de 15 % à 98 %, et tout particulièrement de 20 % à 50 % de monomère(s) à insaturation éthylénique ayant au moins une fonction carboxylique,

b. de 0 % à 83 %, et tout particulièrement de 47 % à 77 % d'autre(s) monomère(s) à insaturation éthylénique démuni(s) de fonction carboxylique,

c. de 2 % à 18 % et tout particulièrement de 3 % à 10 % de monomère spécial.

d. de 0 % à 5 % et tout particulièrement de 0 % à 3 % de monomère(s) possédant au moins deux insaturations éthyléniques.

Le total des constituants (a), (b), (c) et (d) étant égal à 100.

L'invention concerne également les compositions aqueuses chargées et/ou pigmentées contenant le copolymère selon l'invention. Les compositions aqueuses chargées et/ou pigmentées sont plus particulièrement celles qui, outre le copolymère selon l'invention, contiennent une charge minérale telle que le carbonate de calcium, les argiles, les oxydes de fer, les silico-aluminates de sodium ou zéolithes et/ou un ou plusieurs colorants et éventuellement un liant naturel ou synthétique ainsi qu'éventuellement d'autres constituants comme des dispersants, des agents de coalescence, des biocides, des tensioactifs, des antimousses.

Parmi toutes ces compositions aqueuses, contenant le copolymère selon l'invention, on peut par exemple citer les compositions cosmétiques, les pâtes d'impression textile, les suspensions aqueuses de zéolithes, les fluides de forage en particulier à l'eau, les formulations de crème à récurer, les formulations de détergence, les peintures et autres compositions de revêtement.

La portée et l'intérêt de l'invention seront mieux perçus grâce aux exemples suivants qui ne sauraient être limitatifs.

**Exemple 1 :**

Afin de comparer l'efficacité à l'épaississement sous bas gradient de cisaillement de différents épaississants, on prépare des solutions aqueuses de polymères à 0,5 % en poids sec puis on les neutralise à pH = 8,5 à l'aide d'ammoniaque à 28 % avant de les laisser au repos 24 heures.

Au bout de ces 24 heures les viscosités apparentes de ces gels sont déterminées à 25°C à l'aide du viscosimètre Rhéomat 115 commercialisé par la société CONTRAVES et équipé du mobile 145 et pour les taux de cisaillement 0,1 et 0,5 $s^{-1}$.

Dans tous les essais de l'exemple, les copolymères étudiés sont constitués de :

a. 36 % en poids d'acide méthacrylique,
b. 55 % en poids d'acrylate d'éthyle,
c. 9 % en poids du monomère spécial de formule générale (I).

Ainsi, sont testés pour :

L'essai n°1 :

un copolymère selon l'art antérieur dont le monomère spécial de formule générale (I) est un hémimaléate comprenant 25 motifs d'oxyde d'éthylène et un radical R' alkyle linéaire constitué de 12 atomes de carbone.

L'essai n°2 :

Un copolymère selon l'art antérieur dont le monomère spécial de formule générale (I) est un hémimaléate comprenant 25 motifs d'oxyde d'éthylène et un radical R' alkyle linéaire constitué de 16 à 18 atomes de carbone.

L'essai n°3 :

Un copolymère selon l'art antérieur dont le monomère spécial de formule générale (I) est un hémimaléate comprenant 25 motifs d'oxyde d'éthylène et un radical R' alkyle constitué de 22 atomes de carbone.

L'essai n°4 :

Un copolymère selon l'invention dont le monomère spécial de formule générale (I) est un hémimaléate comprenant 25 motifs d'oxyde d'éthylène et un radical R' alkyle constitué de 32 atomes de carbone.

L'essai n°5 :

Un copolymère selon l'invention dont le monomère spécial de formule générale (I) est un hémimaléate comprenant 25 motifs d'oxyde d'éthylène et un radical R' alkyle constitué de 34 atomes de carbone.

L'essai n°6 :

Un copolymère selon l'invention dont le monomère spécial de formule générale (I) est un hémimaléate comprenant 25 motifs d'oxyde d'éthylène et un radical R' alkyle constitué de 36 atomes de carbone.

L'essai n°7 :

Un copolymère selon l'art antérieur dont le monomère spécial de formule générale (I) est un méthacrylate comprenant 25 motifs d'oxyde d'éthylène et un radical R' alkyle linéaire constitué de 12 atomes de carbone.

L'essai n°8 :

Un copolymère selon l'art antérieur dont le monomère spécial de formule générale (I) est un méthacrylate comprenant 25 motifs d'oxyde d'éthylène et un radical R' alkyle linéaire constitué de 16 à 18 atomes de carbone.

L'essai n°9 :

Un copolymère selon l'art antérieur dont le monomère spécial de formule générale (I) est un méthacrylate comprenant 25 motifs d'oxyde d'éthylène et un radical R' alkyle constitué de 22 atomes de carbone.

L'essai n° 10 :

Un copolymère selon l'invention dont le monomère spécial de formule générale (I) est un méthacrylate comprenant 25 motifs d'oxyde d'éthylène et un radical R' alkyle constitué de 32 atomes de carbone.

L'essai n° 11 :

Un copolymère selon l'invention dont le monomère spécial de formule générale (I) est un méthacrylate comprenant 25 motifs d'oxyde d'éthylène et un radical R' alkyle constitué de 34 atomes de carbone.

L'essai n° 12 :

Un copolymère selon l'invention dont le monomère spécial de formule générale (I) est un méthacrylate comprenant 25 motifs d'oxyde d'éthylène et un radical R' alkyle constitué de 36 atomes de carbone.

Les résultats des mesures en Pa.s des viscosités apparentes sous les différents taux de cisaillement en s-1 sont rassemblés pour tous les essais dans le tableau I suivant :

TABLEAU I

| | ESSAI N° | MONOMERE DE FORMULE GENERALE I | | | | | | | | TAUX DE CISAILLEMENT EN $S^{-1}$ | |
|---|---|---|---|---|---|---|---|---|---|---|---|
| | | m | n | p | q | $R_1$ | $R_2$ | R | R' | 0,1 | 0,5 |
| | | | | | | | | | | η en Pa.s | η en Pa.s |
| A.A. | 1 | 0 | 25 | 0 | 1 | - | - | hémimaléate | alkyle en C 12 | 5,75 | 1,15 |
| A.A. | 2 | 0 | 25 | 0 | 1 | - | - | hémimaléate | céto-stéaryle | 6,23 | 1,25 |
| A.A. | 3 | 0 | 25 | 0 | 1 | - | - | hémimaléate | alkyle en C 22 | 5,27 | 1,92 |
| INV. | 4 | 0 | 25 | 0 | 1 | - | - | hémimaléate | alkyle en C 32 | 54 | 13 |
| INV. | 5 | 0 | 25 | 0 | 1 | - | - | hémimaléate | alkyle en C 34 | 49 | 13 |
| INV. | 6 | 0 | 25 | 0 | 1 | - | - | hémimaléate | alkyle en C 36 | 24 | 7 |
| A.A. | 7 | 0 | 25 | 0 | 1 | - | - | méthacrylate | alkyle en C 12 | 0,48 | 0,19 |
| A.A. | 8 | 0 | 25 | 0 | 1 | - | - | méthacrylate | céto-stéaryle | 42,2 | 14,5 |
| A.A. | 9 | 0 | 25 | 0 | 1 | - | - | méthacrylate | alkyle en C 22 | 40 | 12,3 |
| INV. | 10 | 0 | 25 | 0 | 1 | - | - | méthacrylate | alkyle en C 32 | 115 | 37,5 |
| INV. | 11 | 0 | 25 | 0 | 1 | - | - | méthacrylate | alkyle en C 34 | 73,3 | 19,4 |

(suite)

| | ESSAI N° | MONOMERE DE FORMULE GENERALE I | | | | | | | | TAUX DE CISAILLEMENT EN $S^{-1}$ | |
|---|---|---|---|---|---|---|---|---|---|---|---|
| | | m | n | p | q | $R_1$ | $R_2$ | R | R' | 0,1 | 0,5 |
| | | | | | | | | | | η en Pa.s | η en Pa.s |
| INV. | 12 | 0 | 25 | 0 | 1 | - | - | méthacrylate | alkyle en C 36 | 56 | 15 |
| η = viscosité apparente A.A. = ART ANTERIEUR<br>INV. = INVENTION | | | | | | | | | | | |

La lecture du tableau I permet de constater que pour les essais n° 4 à 6 et n° 10 à 12, l'épaississement du gel à 0,5 % en poids sec de polymère est nettement supérieur pour les copolymères selon l'invention constitués d'acide méthacrylique, d'acrylate d'éthyle et d'un monomère oxyalkylé à insaturation éthylénique et terminé par une chaîne grasse hydrophobe, de formule générale (I) dans laquelle R' représente le radical hydrophobe ayant au moins 31 atomes de carbone.

**Exemple 2 :**

Cet exemple concerne l'épaississement d'une base de shampooing contenant du lauryl éther sulfate d'ammonium et des silicones.

Mode opératoire :

La base à épaissir est introduite dans un flacon en verre et mise en agitation très lente à l'aide d'un système d'agitation du type Rayneri.
On pèse successivement et on introduit :

- la base du shampooing à épaissir : 250 g,
- l'eau de qualité industrielle : 25 g,
- puis l'épaississant à tester (contenant 30 % en masse de matières actives) : 5 g.

Le pH est réglé à 6,5 à l'aide de triéthanolamine.
Il est nécessaire d'obtenir un mélange en fin de formulation qui soit convenablement épaissi et qui soit également parfaitement limpide et transparent.
La mesure de la viscosité apparente est effectuée à l'aide d'un viscosimètre Brookfield du type RVT équipé du module adapté à 20°C.
Les lectures sont effectuées à 10 tours/minute et 100 tours/minute immédiatement après la réalisation du mélange et 48 heures après.
Les différents copolymères épaississants testés sont pour :

L'essai n° 13

un copolymère selon l'art antérieur constitué de :

a. 36 % en poids d'acide méthacrylique,
b. 55 % en poids d'acrylate d'éthyle,
c. 9 % en poids d'un méthacrylate comprenant 50 motifs d'oxyde d'éthylène et un radical R' alkyle linéaire constitué de 16 à 18 atomes de carbone.

L'essai n°14

un copolymère selon l'art antérieur constitué de :

a. 36 % en poids d'acide méthacrylique,

b. 55 % en poids d'acrylate d'éthyle,
c. 9 % en poids d'un méthacrylate comprenant 20 motifs d'oxyde d'éthylène et un radical R' alkyle constitué de 22 atomes de carbone.

<u>L'essai n° 15</u>

un copolymère selon l'art antérieur constitué de :

a. 36 % en poids d'acide méthacrylique,
b. 55 % en poids d'acrylate d'éthyle,
c. 9 % en poids d'un méthacrylate comprenant 50 motifs d'oxyde d'éthylène et un radical R' alkylaryle nonylphényle.

<u>L'essai n°16</u>

un copolymère selon l'art antérieur constitué de :

a. 36 % en poids d'acide méthacrylique,
b. 55 % en poids d'acrylate d'éthyle,
c. 9 % en poids d'un méthacrylate comprenant 35 motifs d'oxyde d'éthylène et un radical R' alkyle constitué de 22 atomes de carbone.

<u>L'essai n° 17</u>

un copolymère selon l'invention constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 32 atomes de carbone.

<u>L'essaie n°18</u>

un copolymère selon l'invention constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 34 atomes de carbone.

<u>L'essai n° 19</u>

un copolymère selon l'invention constitué de :

a. 36 % en poids d'acide méthacrylique,
b. 55 % en poids d'acrylate d'éthyle,
c. 9 % en poids d'un méthacrylate comprenant 15 motifs d'oxyde d'éthylène et un radical R' alkyle constitué de 34 atomes de carbone.

Les résultats des mesures des viscosités Brookfield des différents essais sont rassemblés dans le tableau II suivant.

TABLEAU II

| | ESSAI N° | MONOMERE DE FORMULE GENERALE I | | | | | | | η IMMEDIATE EN mPa.s | | η 48 HEURES EN mPa.s | | ASPECT DU GEL |
|---|---|---|---|---|---|---|---|---|---|---|---|---|---|
| | | m | n | p | q | R1 | R2 | R' | 10 t/mn | 100 t/mn | 10 t/mn | 100 t/mn | |
| A.A. | 13 | 0 | 50 | 0 | 1 | - | - | céto-stéaryle | 600 | 560 | 1300 | 1050 | trouble |
| A.A. | 14 | 0 | 20 | 0 | 1 | - | - | alkyle en C 22 | 400 | 420 | 500 | 500 | opaque |
| A.A. | 15 | 0 | 50 | 0 | 1 | - | - | nonyl-phenyle | 500 | 520 | 700 | 590 | trouble |
| A.A. | 16 | 0 | 35 | 0 | 1 | - | - | alkyle en C 22 | 600 | 590 | 1000 | 800 | trouble |
| INV. | 17 | 0 | 25 | 0 | 1 | - | - | alkyle en C 32 | 800 | 60 | 1100 | 980 | limpide |
| INV. | 18 | 0 | 25 | 0 | 1 | - | - | alkyle en C 34 | 800 | 740 | 1100 | 960 | limpide |
| INV. | 19 | 0 | 15 | 0 | 1 | - | - | alkyle en C 34 | 600 | 540 | 1300 | 1060 | limpide |
| η = viscosité Brookfield A.A. = ART ANTERIEUR INV. = INVENTION | | | | | | | | | | | | | |

Cette base de shampooing est considérée comme bonne si sa viscosité Brookfield à 10 tours/minute et 20°C est supérieure à 1 000 mPa.s après 48 heures et si sa formulation est limpide.

La lecture du tableau II nous permet de distinguer que seuls les essais n°17 à 19 selon l'invention constituent une bonne base de shampooing.

**Exemple 3**

Cet exemple concerne l'épaississement et la formulation de pâte d'impression textile.

Dans ce but, on introduit sous agitation dans un flacon en verre 441 g d'eau et 17,5 g d'épaississant à 30 % de matière active à tester ainsi que 4 g d'antimousse.

L'agitation effectuée à l'aide d'un agitateur type Rayneri est maintenue constante pendant un quart d'heure, période pendant laquelle le pH est ajusté à 8 à l'aide d'une solution d'ammoniaque à 30 %.

Puis on rajoute 50 g d'un latex de type styrène-butadiène thermoréticulable au moyen de mélamine formol que l'on homogénéise par agitation pendant 10 minutes avant d'incorporer 15 g d'un colorant bleu marine Néoprint SOFAT.

Ensuite, 15 g de tensioactif non ionique de type nonylphénol possédant 4 motifs d'oxyde d'éthylène sont ajoutés à cette composition. L'agitation dure 10 minutes.

Puis on mesure la viscosité Brookfield de la pâte d'impression textile à 20°C et 10 tours/minute au moyen d'un viscosimètre Brookfield type RVT équipé du mobile adéquat.

L'essai n°20

Dans cet essai, l'épaississant testé est un copolymère selon l'invention constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 32 atomes de carbone.

L'essai n°21

l'épaississant testé dans cet essai est un copolymère selon l'invention constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R' alkyle constitué de 32 atomes de carbone.

L'essai n°22 :

l'épaississant testé dans cet essai est un copolymère selon l'invention constitué de :

a. 36 % en poids d'acide méthacrylique,
b. 48 % en poids d'acrylate d'éthyle,
c. 16 % en poids d'un hémimaléate comprenant 25 motifs d'oxyde d'éthylène et un radical R' alkyle constitué de 32 atomes de carbone.

L'essai n°23 :

l'épaississant testé dans cet essai est un copolymère selon l'invention constitué de :

a. 43 % en poids d'acide méthacrylique,
b. 51 % en poids d'acrylate d'éthyle,
c. 3 % en poids d'un méthacrylate comprenant 25 motifs d'oxyde d'éthylène et un radical R' alkyle constitué de 32 atomes de carbone,
d. 3 % en poids d'un mélange équipondéral de méthylène-bis-acrylamide et de diméthacrylate d'éthylène glycol.

Les résultats obtenus sont rassemblés dans le tableau III suivant.

TABLEAU III

| | ESSAI N° | MONOMERE DE FORMULE GENERALE I | | | | | | | | η EN mPa.s 10 t/mn | RENDU DE COULEUR | REPARTITION PIGMENTAIRE |
|---|---|---|---|---|---|---|---|---|---|---|---|---|
| | | m | n | p | q | R1 | R2 | R | R' | | | |
| INV. | 20 | 0 | 25 | 0 | 1 | - | - | méthacrylate | alkyle à 32 C | 30000 | BON | BONNE |
| INV. | 21 | 0 | 25 | 0 | 1 | - | - | hémimaléate | alkyle à 32 C | 25000 | BON | BONNE |
| INV. | 22 | 0 | 25 | 0 | 1 | - | - | hémimaléate | alkyle à 32 C | 40000 | BON | BONNE |
| INV. | 23 | 0 | 25 | 0 | 1 | - | - | méthacrylate | alkyle à 32 C | 45000 | BON | BONNE |

η = viscosité Brookfield INV. = INVENTION

Une pâte d'impression textile est utilisable si, d'une part sa viscosité Brookfield mesurée à 10 tours/minute et 20°C est supérieure ou égale à 25 000 m.Pas et si, d'autre part, ladite pâte, une fois appliquée sur un support textile présente, après séchage de quelques minutes à l'étuve à 100°C, un rendu de couleur et une répartition pigmentaire acceptable.
La lecture du tableau III permet de constater que les copolymères selon l'invention des essais n°20 à 23 donnent de bonnes formulations en pâte d'impression textile.

**Exemple 4**

Cet exemple concerne l'utilisation des copolymères selon l'invention comme stabilisant des suspensions aqueuses de zéolithes.
Dans ce but, la Demanderesse utilise dans tous les essais la même suspension aqueuse de zéolithe de type 4A contenant 52,5 % de matière sèche.
Cette suspension d'aspect très fluide, lorsqu'elle est maintenue en agitation, sédimente en quelques heures, si elle ne contient aucun agent stabilisant, et se sépare en deux phases. L'une en un liquide surnageant et l'autre conduisant à un sédiment très dur impossible à remettre en suspension sans moyen mécanique puissant.
Les tailles de particules de zéolithe sont comprises entre 1 et 10 $\mu$m et la suspension a un pH alcalin supérieur à 12.

Mode opératoire :

La suspension est fractionnée en échantillons de 240 g. Chaque fraction est conservée dans un flacon en verre de 230 ml muni d'un couvercle métallique hermétique.
Chaque flacon ne servira qu'à un seul essai de stabilisation. Pour chaque essai, le contenu du pot est remis en suspension fluide et homogène à l'aide d'un système d'agitation du type Rayneri, avant d'incorporer 1 g d'agent stabilisant à tester, ce qui correspond à 0,13 % en poids sec d'agent par rapport à la masse totale de la suspension. L'agitation est maintenue pendant 15 minutes de façon à obtenir un mélange intime de la suspension à stabiliser et de l'agent testé. Après ce temps, la rhéologie du système est déterminée à l'aide d'un viscosimètre Brookfield type RVT équipé d'un module adapté. La viscosité apparente To est mesurée à 20°C. Chaque lecture est effectuée à 10 tours/minute et 100 tours/minute après 2 minutes de rotation. Les récipients contenant la suspension ainsi stabilisée, munis de leurs couvercles hermétiques, sont laissés au repos à température ambiante pour un cycle de vieillissement statique de quatre jours.
Après ce laps de temps, on détermine pour chaque essai la stabilité puis la rhéologie de la suspension.

**a. La stabilité**

Elle est déterminée par la mesure à l'aide d'une réglette graduée de la hauteur du sédiment au fond du flacon en verre. La suspension aqueuse de zéolithe est considérée comme stable si la hauteur du dépôt est inférieure ou égale à 1 mm.

**b. La rhéologie**

La rhéologie du système se détermine par la mesure de la viscosité apparente de la suspension à 20°C, à l'aide d'un viscosimètre Brookfield type RVT équipé du module adapté. Les lectures sont faites à 10 tours/minute et 100 tours/minute après deux minutes de rotation et sont notées T4J.
La suspension aqueuse est considérée comme pompable lorsque la viscosité apparente mesurée à 10 tours/minute est inférieure ou égale à 2 500 mPa.s (cP).
La mesure de rhéologie (viscosité Brookfield) effectuée après 4 jours de stockage est répétée après un deuxième cycle de vieillissement statique de 40 jours à température ambiante et notée T40J.

L'essai n°24 :

Il constitue l'essai témoin et aucun agent stabilisant n'a été ajouté.
Par contre, les différents agents stabilisants sont pour :

L'essai n°25 :

un copolymère selon l'invention constitué de :

a. 36 % en poids d'acide méthacrylique,
b. 58 % en poids d'acrylate d'éthyle,
c. 6 % en poids d'un hémimaléate comprenant 25 motifs d'oxyde d'éthylène et un radical R' alkyle constitué de 32 atomes de carbone.

L'essai n°26 :

un copolymère selon l'invention constitué de :

a. 36 % en poids d'acide méthacrylique,
b. 55 % en poids d'acrylate d'éthyle,
c. 9 % en poids d'un hémimaléate comprenant 20 motifs d'oxyde d'éthylène et un radical R' alkyle constitué de 36 atomes de carbone.

Les résultats des mesures de viscosité apparente Brookfield To, T4J, T40J et de hauteur de dépôt des divers essais sont rassemblés dans le tableau IV suivant.

TABLEAU IV

| | ESSAI N° | MONOMERE DE FORMULE GENERALE I | | | | | | | | TEMPS | | | |
|---|---|---|---|---|---|---|---|---|---|---|---|---|---|
| | | | | | | | | | | $T_0$ | $T_{4J}$ | | $T_{40J}$ |
| | | m | n | p | q | $R_1$ | $R_2$ | R | R' | η en mPa.s | η en mPa.s | D. | η en mPa.s |
| T. | 24 | - | - | - | - | - | - | - | - | 80 - 75 | INFINIE | 36 mm | INFINIE |
| INV. | 25 | 0 | 25 | 0 | 1 | - | - | hémimaléate | alkyle à 32 C | 260 - 150 | 1300 - 350 | < 1 mm | 1100 - 290 |
| INV. | 26 | 0 | 25 | 0 | 1 | - | - | hémimaléate | alkyle à 36 C | 300 - 130 | 1300 - 360 | < 1 mm | 1000 - 250 |

η en mPa.s = viscosité Brookfield en mPa.s mesurée à 10 t/mn puis 100 t/mn T. = TEMOIN
D. = hauteur de dépôt en millimètres INV. = INVENTION

La lecture du tableau IV permet de constater que les suspensions des essais n°25 et 26 selon l'invention possèdent après 4 et 40 jours à température ambiante une viscosité Brookfield (T4J, T40J) inférieure ou égale à 2 500 mPa.s à 10 tours/minute et 20°C ainsi qu'une hauteur de dépôt inférieure ou égale à 1 mm.

Ainsi, les copolymères selon l'invention sont de bons agents de stabilisation des suspensions aqueuses de zéolithe.

**Exemple 5**

Cet exemple illustre l'utilisation des copolymères selon l'invention comme additif de boues de forage à l'eau de mer et plus particulièrement comme modificateur de rhéologie.

Ainsi, pour chaque essai, on prépare trois solutions contenant chacune 35 g d'argile à moyen rendement de gonflement sélectionnée par l'OCMA (Oil Company Materials Association) et 1 g de $NaHCO_3$ dans 350 $cm^3$ d'eau de mer.

On agite 20 minutes et on ajoute pour les essais autres que le témoin 5, 10 et 15 g de copolymère sec à tester par litre dans les trois solutions et on amène le pH à 9 à l'aide de soude à 35 %.

Après 24 heures de repos dans les récipients fermés, on agite chaque suspension pendant 5 minutes et on amène éventuellement le pH à 9 à l'aide de soude à 35 % avant de mesurer la viscosité apparente de chaque suspension au viscosimètre Fann.

La norme API (American Petroleum Institute), définit, pour les fluides de forage, la viscosité apparente (Va) correspondant à un taux de cisaillement de 1 020 $s^{-1}$, soit une vitesse du rotor du viscosimètre Fann de 600 tours/minute.

Lorsqu'elle est exprimée en centipoises ou milliPascal.seconde, la viscosité apparente est donnée par l'expression :

$$Va = \frac{\text{lecture Fann à 600 tours/minute}}{2}$$

L'essai n°27 constitue l'essai témoin, c'est-à-dire l'essai, qui, sans ajout de copolymère, permet de mesurer la viscosité apparente de la boue après 24 heures de repos.

Dans l'essai n°28, le copolymère testé est un copolymère selon l'invention constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R' alkyle constitué de 32 atomes de carbone.

Dans l'essai n°29, le copolymère selon l'invention est constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R' alkyle constitué de 34 atomes de carbone.

Les résultats des mesures de viscosité apparente Fann des différents essais sont rassemblés dans le tableau V suivant.

TABLEAU V

| | ESSAI N° | MONOMERE DE FORMULE GENERALE I | | | | | | | | Dose (*) | LECTURE 600 Tours / Mn | VISCOSITE APPARENTE EN mPa.s |
|---|---|---|---|---|---|---|---|---|---|---|---|---|
| | | m | n | p | q | R 1 | R 2 | R | R' | | | |
| T. | 27 | - | - | - | - | - | - | - | - | 0 | 10 | 5 |
| INV. | 28 | 0 | 25 | 0 | 1 | - | - | hémimaléate | alkyle à 32 C | 5 | 85 | 42,5 |
| | | | | | | | | | | 10 | 162 | 81 |
| | | | | | | | | | | 15 | 220 | 110 |
| INV. | 29 | 0 | 25 | 0 | 1 | - | - | hémimaléate | alkyle à 34 C | 5 | 106 | 53 |
| | | | | | | | | | | 10 | 200 | 100 |
| | | | | | | | | | | 15 | 274 | 137 |

*(*) exprimée en g d'additif sec pour un litre de boue de forage à l'eau de mer.*
T. = TEMOIN INV. = INVENTION

Les suspensions d'argile sont utilisables en boue de forage à l'eau de mer si leur viscosité apparente Fann est supérieure à 25 mPa.s (cP).

La lecture du tableau V permet de constater que les boues de forage à l'eau de mer des essais n°28 et 29 ont des viscosités apparentes supérieures à 25 mPa.s.

Ainsi, les copolymères selon l'invention modifient la structure de la suspension initiale d'argile dans de l'eau de mer et procurent à cette suspension d'argile de charge à moyen rendement de gonflement une rhéologie utilisable dans les boues de forage à l'eau de mer.

**Exemple 6 :**

Cet exemple concerne l'épaississement d'une base de crème à récurer contenant un mélange de tensio-actif non ionique et du carbonate de calcium de diamètre moyen égal à 30μm commercialisé par la société OMYA sous le nom de Durcal 40.

Mode opératoire :

Dans un flacon en verre de 1 000 ml équipé d'un système d'agitation du type Rayneri, on introduit successivement et sous agitation :

- 330 g d'eau brute,
- 2,25 g d'un dispersant acrylique commercialisé par la société COATEX sous le nom de COATEX P90,
- et 375 g du carbonate de calcium Durcal 40.

La suspension aqueuse de carbonate de calcium ainsi obtenue est alors alcalinisée jusqu'à pH = 9,5 à l'aide d'ammoniaque à 28 %.
Une fois le pH atteint on incorpore, toujours sous agitation, 10 g du copolymère à tester contenant 30 % de matière active et 3 g du tensio-actif non ionique du type nonylphénol condensé avec 10 molécules d'oxyde d'éthylène.
Après 5 minutes d'agitation, on stoppe celle-ci et on estime la sédimentation immédiate ($T_O$) à l'aide d'une spatule introduite dans la base de crème à récurer. On peut alors distinguer la sédimentation particulièrement rapide par le fait que la spatule rencontre une résistance à la pénétration au fond du flacon.
Les bases de crème à récurer sont alors laissées au repos pendant 48 heures.
Au bout de ces 48 heures, on mesure d'une part la viscosité apparente Brookfield à 20°C et à 10 tours/minute et 100 tours/minute au moyen d'un viscosimètre Brookfield type RVT équipé du module adéquat et d'autre part la sédimentation (T48H) du mélange estimé de la même façon que la sédimentation immédiate.

Les différents copolymères testés sont pour :

L'essai n°30 :

un copolymère selon l'art antérieur constitué de :

a. 36 % en poids d'acide méthacrylique,
b. 55 % en poids d'acrylate d'éthyle
c. 9 % en poids d'un hémimaléate comprenant 25 motifs d'oxyde d'éthylène et un radical R' alkyle linéaire constitué de 16 à 18 atomes de carbone.

L'essai n°31 :

un copolymère selon l'art antérieur constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle linéaire constitué de 16 à 18 atomes de carbone.

L'essai n°32 :

un copolymère selon l'art antérieur constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R'alkyle constitué de 22 atomes de carbone.

L'essai n°33 :

un copolymère selon l'art antérieur constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 22 atomes de carbone.

L’essai n°34 :

un copolymère selon l’invention constitué de :

a. 36 % en poids d’acide méthacrylique,
b. 55 % en poids d’acrylate d’éthyle,
c. 9 % en poids d’un méthacrylate comprenant 25 motifs d’oxyde d’éthylène et un radical R’ alkyle constitué de 32 atomes de carbone.

L’essai n°35

un copolymère selon l’invention constitué de :

a. 36 % en poids d’acide méthacrylique,
b. 55 % en poids d’acrylate d’éthyle,
c. 9 % en poids d’un méthacrylate comprenant 15 motifs d’oxyde d’éthylène et un radical R’ alkyle constitué de 36 atomes de carbone.

Les résultats des mesures de viscosité apparente Brookfield à 10 tours/minute et 100 tours/minute à 20°C après 48 heures de repos ainsi que ceux de sédimentation (To) et de sédimentation (T48H) après 48 heures de repos des différents essais sont rassemblés dans le tableau VI suivant.

TABLEAU VI

| | ESSAI N° | MONOMERE DE FORMULE GENERALE I | | | | | | | | SEDIMENTATION $T_O$ | $\eta$ 48H en mPa.s | | SEDIMENTATION $T_{48H}$ |
|---|---|---|---|---|---|---|---|---|---|---|---|---|---|
| | | m | n | p | q | $R_1$ | $R_2$ | R | R' | | 10 t/mn | 100 t/mn | |
| A.A. | 30 | 0 | 25 | 0 | 1 | - | - | hémimaléate | céto-stéaryle | OUI | < 300 | - | OUI |
| A.A. | 31 | 0 | 25 | 0 | 1 | - | - | méthacrylate | céto-stéaryle | OUI | < 300 | - | OUI |
| A.A. | 32 | 0 | 25 | 0 | 1 | - | - | hémimaléate | alkyle à 22 C | OUI | < 300 | - | OUI |
| A.A. | 33 | 0 | 25 | 0 | 1 | - | - | méthacrylate | alkyle à 22 C | OUI | < 300 | - | OUI |
| INV. | 34 | 0 | 25 | 0 | 1 | - | - | méthacrylate | alkyle à 32 C | NON | 13 000 | 6000 | NON |
| INV. | 35 | 0 | 15 | 0 | 1 | - | - | méthacrylate | alkyle à 36 C | NON | 20 000 | 10 000 | NON |

$\eta$ = viscosité Brookfield A.A. : ART ANTERIEUR
INV. = INVENTION

Une base de crème à récurer est considérée comme bonne si, après 48 heures de stockage, le système épaissi en présence de tensio-actif non ionique présente une viscosité apparente Brookfield supérieure à 3 000 mPa.s à 10 tours/minute et aucune sédimentation au bout d'au moins 48 heures.
La lecture du tableau VI permet de constater que seuls les essais n°34 et 35 selon l'invention donnent des formulations à viscosité apparente élevée et sans sédimentation.

**Exemple 7** :

Cet exemple concerne l'utilisation du copolymère selon l'invention comme agent antisédimentation de suspension aqueuse de carbonate de calcium grossier.

Mode opératoire :

Dans un bécher de 1 000 ml équipé d'un système d'agitation du type Rayneri, on introduit sous agitation et dans l'ordre suivant :

- l'eau,
- le dispersant acrylique de poids moléculaire 7 000 commercialisé par la société COATEX sous le nom de COATEX P90 à 40 % de matière active,
- le copolymère à tester,
- puis un mélange de carbonate de calcium grossier provenant du gisement de Salses dont 30 parties sont constituées de carbonate de calcium dont 29 % des particules sont inférieures à 2μm et commercialisé par la société OMYA sous le nom de Durcal 5 et dont 70 parties sont constituées de carbonate de calcium dont 25 % des particules sont inférieures à 12 μm et commercialisé par la société OMYA sous le nom de Calibrite SL.

La suspension aqueuse ainsi réalisée est alors maintenue sous forte agitation pendant 30 minutes.
L'agitation stoppée, on mesure à l'aide d'un viscosimètre Brookfield type RVT équipé du module adapté la viscosité apparente Brookfield To de la suspension à 20°C et à 10 tours/minute et 100 tours/minute.
Cette mesure effectuée, la suspension est transvasée dans un pot en plastique fermé de 500 ml à ouverture totale, puis stockée au repos à 25°C.
Au bout de 8 jours de stockage, l'aspect de la suspension est déterminé par introduction d'une spatule au fond du pot.
Les échantillons présentant une très forte sédimentation sont considérés très mauvais et notés 0. Les échantillons présentant une forte sédimentation sont considérés mauvais et notés 1.
De même, ceux présentant une sédimentation très collante sont considérés comme médiocres et notés 2 tandis que ceux ayant une sédimentation peu importante mais collante sont considérés comme moyens et notés 3. La notation 4 correspond aux échantillons estimés comme bons car ne présentant aucune sédimentation et un aspect de gel très épais alors que la notation 5 est réservée aux très bons échantillons n'ayant aucune sédimentation et un aspect de gel épais.
Cet aspect de la suspension est également contrôlé de la même manière après 15 et 30 jours de stockage à 25°C.
Au bout de 30 jours de stockage et après le contrôle de l'aspect de la suspension, on soumet ladite suspension à une agitation mécanique pendant deux minutes puis on mesure la viscosité apparente Brookfield T30J à 20°C et à 10 tours/minute et 100 tours/minute au moyen d'un viscosimètre Brookfield type RVT équipé du module adapté.
Pour tous les essais, on utilise deux béchers dans lesquels on introduit pour tous les essais la même quantité d'eau, de dispersant et de carbonate de calcium de manière à obtenir une suspension aqueuse de carbonate de calcium à 75 % en poids de matière sèche et comprenant 0,035 % en poids sec de dispersant par rapport au poids sec de carbonate de calcium.
Le copolymère à tester est lui, introduit dans tous les essais suivant deux doses : dans le premier bécher à raison de 0,05 % en poids sec par rapport au poids sec de carbonate de calcium et dans le deuxième bécher à raison de 0,1 % en poids sec par rapport au poids sec de carbonate de calcium.

L'essai n°36 :

Il constitue l'essai témoin et ne comprend aucun agent antisédimentation.
Par contre, les différents agents de sédimentation sont pour :

L'essai n°37 :

un copolymère selon l'art antérieur constitué de :

a. 40,4 % en poids d'acide méthacrylique,
b. 59 % en poids d'acrylate d'éthyle,
c. 0,6 % en poids de réticulant.

L'essai n°38 :

un copolymère selon l'art antérieur constitué de :

a. 99,2 % en poids d'acide acrylique,
b. 0,8 % en poids de réticulant.

L'essai n°39 :

un copolymère selon l'art antérieur constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle linéaire constitué de 16 à 18 atomes de carbone.

L'essai n°40 :

un copolymère selon l'invention constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R' alkyle constitué de 32 atomes de carbone.

L'essai n°41 :

un copolymère selon l'invention constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 32 atomes de carbone.

L'essai n°42 :

un copolymère selon l'invention constitué de :

a. 36 % en poids d'acide méthacrylique,
b. 55 % en poids d'acrylate d'éthyle,
c. 9 % en poids d'un hémimaléate comprenant 25 motifs d'oxyde d'éthylène et un radical R' alkyle constitué de 36 atomes de carbone.

Les résultats des mesures en mPa.s des viscosités Brookfield (To, T30J) à 10 tours/minute et 100 tours/minute et 20°C ainsi que l'aspect des suspensions après 8 jours, 15 jours et 30 jours de stockage au repos à 25°C sont rassemblés pour tous les essais dans le tableau VII suivant.

TABLEAU VII

| | ESSAI N° | MONOMERE DE FORMULE GENERALE I | | | | | | | | Dose (*) | η $T_O$ En mPa.s | ASPECT DU GEL APRES | | | η$T_{30J}$ En mPa.s |
|---|---|---|---|---|---|---|---|---|---|---|---|---|---|---|---|
| | | m | n | p | q | $R_1$ | $R_2$ | R | R' | | | 8 jours | 15jours | 30 jours | |
| T. | 36 | - | - | - | - | - | - | - | - | 0 | 3000 - 1500 | 0 | 0 | 0 | INFINIE |
| A.A. | 37 | - | - | - | - | - | - | - | - | 0,05<br>0,1 | 5300 - 3400<br>4800 - 2800 | 3<br>3 | 1<br>2 | 0<br>0 | INFINIE<br>INFINIE |
| A.A. | 38 | - | - | - | - | - | - | - | - | 0,05<br>0,1 | 7200 - 3600<br>6200 - 2600 | 4<br>4 | 3<br>3 | 2<br>2 | 6800 - 3200<br>6300 - 2700 |
| A.A. | 39 | 0 | 25 | 0 | 1 | - | - | méthacrylate | céto-stéaryle | 0,05<br>0,1 | 600 - 490<br>600 - 540 | 0<br>0 | 0<br>0 | 0<br>0 | INFINIE<br>INFINIE |
| INV. | 40 | 0 | 25 | 0 | 1 | - | - | hémimaléate | alkyle à 32 C | 0,05<br>0,1 | 700 - 450<br>500 - 550 | 4<br>5 | 2<br>4 | 2<br>4 | 1300 - 800<br>1100 - 800 |
| INV. | 41 | 0 | 25 | 0 | 1 | - | - | méthacrylate | alkyle à 32 C | 0,05<br>0,1 | 1400-620<br>2000 - 930 | 5<br>5 | 4<br>5 | 4<br>5 | 1800 - 800<br>2600 - 1200 |
| INV. | 42 | 0 | 25 | 0 | 1 | - | - | hémimaléate | alkyle à 36 C | 0,05<br>0,1 | 400 - 390<br>800 - 800 | 4<br>5 | 3<br>4 | 3<br>4 | 1000 - 1000<br>1800 - 1800 |

(*) exprimée en pourcentage d'additif sec par rapport au poids sec de la suspension T. = TEMOIN
η = viscosité Brookfield mesurée à 10 t/mn puis 100 t/mn A.A. = ART ANTERIEUR
INV. = INVENTION

La lecture du tableau VII permet de constater que seuls les essais n°40 à 42 selon l'invention ont un aspect bon ou très bon (notation 4 ou 5) pour une dose de 0,1% en poids sec d'agent antisédimentation ainsi que des viscosités Brookfield pas trop élevées.

**Exemple 8** :

Cet exemple concerne l'épaississement sous bas taux de cisaillement de formulation type peinture.

Mode opératoire :

Dans un bécher de 1 000 ml équipé d'un système d'agitation du type Rayneri, on mélange sous agitation :

- 206 g d'eau,
- 3,04 g d'un dispersant acrylique de poids moléculaire 7 000 à 40 % de matière active commercialisé par la société COATEX sous le nom de COATEX P90,
- 1,96 g d'un biocide commercialisé par la société OMYA sous le nom de Mergal K6N,
- et 0,98 g d'un antimousse commercialisé sous le nom de BYK 34 par la société BYK CHEMIE.

Puis on introduit en pluie dans le mélange agité :

- 40,27 g de dioxyde de titane commercialisé par la société THANN et MULHOUSE sous le nom de RL 68,
- 322,10 g de carbonate de calcium de diamètre moyen égal à 3 μm commercialisé par la société OMYA sous le nom de DURCAL 2,
- et 211,40 g de carbonate de calcium de diamètre moyen égal à 1,5 μm commercialisé par la société OMYA sous le nom d'HYDROCARB.

On disperse alors pendant 20 minutes à l'aide de la turbine crantée de 70 mm de diamètre à une vitesse de 1 500 tours/minute puis on introduit sous agitation :

- 10,02 g de monoéthylène glycol,
- 80,55 g d'eau,
- 80,55 g d'un liant styrène-acrylique commercialisé par la société RHONE-POULENC sous le nom de RHODOPAS DS 910,
  ainsi que :

- 10,02 g de white-spirit.

Cette base de peinture non épaissie terminée, on en mesure la viscosité Brookfield à 0,5 tours/minute et 1 tour/minute à 20°C au moyen d'un viscosimètre Brookfield type RVT équipé du module 1, puis on rajoute le copolymère épaississant à tester à raison de 2,06 % en poids avant d'ajuster le pH à 8,6 à l'aide d'ammoniaque à 28 %.

On mesure alors pour chaque essai la viscosité de la peinture à 0,5 tours/minute et 1 tour/minute à 20°C au moyen d'un viscosimètre Brookfield type RVT équipé du module 6.

L'essai n°43 :

Il constitue l'essai témoin et ne comprend aucun agent épaississant.

L'essai n°44 :

Un copolymère selon l'art antérieur constitué de :

a. 40,4 % en poids d'acide méthacrylique,
b. 59 % en poids d'acrylate d'éthyle,
c. 0,6 % en poids de réticulant.

L'essai n°45 :

Un copolymère selon l'invention constitué de :

a. 36 % en poids d'acide méthacrylique,
b. 55 % en poids d'acrylate d'éthyle,
c. 9 % en poids d'un méthacrylate comprenant 25 motifs d'oxyde d'éthylène et un radical R' alkyle constitué de 32 atomes de carbone.

Les résultats des mesures en Pa.s des viscosités Brookfield à 0,5 tour/minute et 1 tour/minute sont rassemblés dans le tableau VIII suivant.

TABLEAU VIII

| | ESSAI N° | MONOMERE DE FORMULE GENERALE I | | | | | | | | η En Pa.s | |
|---|---|---|---|---|---|---|---|---|---|---|---|
| | | m | n | p | q | R1 | R2 | R | R' | 0,5 t/mn | 1 t/mn |
| T. | 43 | - | - | - | - | - | - | - | - | 0,08 | 0,08 |
| A.A. | 44 | - | - | - | - | - | - | - | - | 500 | 300 |
| INV. | 45 | 0 | 25 | 0 | 1 | - | - | méthacrylate | alkyle en C 32 | 700 | 500 |

η = *viscosité Brookfield* T. = TEMOIN
A.A. = ART ANTERIEUR
INV. = INVENTION

La lecture du tableau VIII permet de constater que la formulation peinture n° 45 selon l'invention donne beaucoup plus de viscosité à faible gradient de vitesse et faible taux de cisaillement que celle de l'essai n°44 selon l'art antérieur.
De plus, la rhéologie conférée par le copolymère épaississant selon l'invention est très proche de celle des "crèmes de peinture" à savoir un aspect très pâteux au repos mais qui se détruit avec une très faible agitation.

## Revendications

1. Compositions aqueuses chargées et/ou pigmentées contenant un copolymère de formule générale :

$$(A)_a\text{---}(B)_b\text{---}(C)_c\text{---}(D)_d$$

dans laquelle :

(A) représente le ou les monomères à insaturation éthylénique et à fonction carboxylique avec "a" le pourcentage pondéral du monomère (A) par rapport au poids total des monomères,
(B) représente le ou les monomères à insaturation éthylénique et sans fonction carboxylique avec "b" le pourcentage pondéral du monomère (B) par rapport au poids total des monomères, "b" pouvant être nul,
(C) représente le monomère oxyalkylé à insaturation éthylénique et terminé par une chaîne grasse hydrophobe possédant au moins 26 atomes de carbone avec "c" le pourcentage pondéral du monomère (C) par rapport au poids total des monomères,
(D) représente le ou les monomères possédant au moins deux insaturations éthyléniques avec "d" le pourcentage pondéral du monomère (D) par rapport au poids total des monomères, "d" pouvant être nul et tel que a + b + c + d = 100 %.

2. Compositions aqueuses chargées et/ou pigmentées selon la revendication 1, **caractérisées en ce que** le copolymère contient, exprimé en pour cent en poids :

(a) de 15 % à 98 %, et tout particulièrement de 20 % à 50 % de monomère(s) à insaturation éthylénique ayant au moins une fonction carboxylique,
(b) de 0 % à 83 %, et tout particulièrement de 47 % à 77 % d'autre(s) monomère(s) à insaturation éthylénique démuni(s) de fonction carboxylique,
(c) de 2 % à 18 % et tout particulièrement de 3 % à 10 % de monomère oxyalkylé à insaturation éthylénique et terminé par une chaîne grasse hydrophobe possédant au moins 26 atomes de carbone,
(d) de 0 % à 5 % et tout particulièrement de 0 % à 3 % de monomère(s) possédant au moins deux insaturations éthyléniques,

le total des constituants (a), (b), (c) et (d) étant égal à 100.

**3.** Compositions aqueuses chargées et/ou pigmentées selon l'une des revendications 1 ou 2, **caractérisées en ce que** le monomère éthylénique carboxylé est choisi dans le groupe constitué par les acides acrylique, méthacrylique et itaconique.

**4.** Compositions aqueuses chargées et/ou pigmentées selon l'une des revendications 1 à 3, **caractérisées en ce que** le monomère à insaturation éthylénique sans fonction carboxylique est choisi parmi les esters acryliques, tels que les acrylate et méthacrylate d'alkyle en $C_1$ à $C_4$.

**5.** Compositions aqueuses chargées et/ou pigmentées selon l'une des revendications 1 à 4, **caractérisées en ce que** le copolymère est obtenu par des procédés de copolymérisation radicalaire en solution, en émulsion directe ou inverse, en suspension ou précipitation dans des solvants, en présence de systèmes catalytiques et d'agents de transfert.

**6.** Compositions aqueuses chargées et/ou pigmentées selon l'une des revendications 1 à 5, **caractérisées en ce qu'**elles contiennent une charge minérale telle que le carbonate de calcium, les argiles, les oxydes de fer, les silico-aluminates de sodium ou zéolithes et/ou un ou plusieurs colorants.

**7.** Compositions aqueuses chargées et/ou pigmentées selon la revendication 6, **caractérisées en ce qu'**elles contiennent un liant naturel ou synthétique ainsi que d'autres constituants comme des dispersants, des agents de coalescence, des biocides, des tensioactifs, des antimousses.

**8.** Compositions aqueuses chargées et/ou pigmentées selon l'une des revendications 1 à 7, **caractérisées en ce qu'**elles sont des compositions cosmétiques.

**9.** Compositions aqueuses chargées et/ou pigmentées selon l'une des revendications 1 à 7, **caractérisées en ce qu'**elles sont des compositions de pâtes d'impression textile.

**10.** Compositions aqueuses chargées et/ou pigmentées selon l'une des revendications 1 à 7, **caractérisées en ce qu'**elles sont des suspensions aqueuses de zéolithes.

**11.** Compositions aqueuses chargées et/ou pigmentées selon l'une des revendications 1 à 7, **caractérisées en ce qu'**elles sont des compositions de fluides de forage en particulier à l'eau.

**12.** Compositions aqueuses chargées et/ou pigmentées selon l'une des revendications 1 à 7, **caractérisées en ce qu'**elles sont des compositions de crème à récurer.

**13.** Compositions aqueuses chargées et/ou pigmentées selon l'une des revendications 1 à 7, **caractérisées en ce qu'**elles sont des formulations de détergence.

**14.** Compositions aqueuses chargées et/ou pigmentées selon l'une des revendications 1 à 7, **caractérisées en ce qu'**elles sont des compositions de peintures et autres compositions de revêtement.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0173109 A
- EP 0013836 A
- EP 0011806 A
- EP 0248612 A
- EP 0216479 A